(19) Europäisches Patentamt
European Patent Office
Office européen des brevets

(11) EP 1 281 710 A1

(12) EUROPEAN PATENT APPLICATION

(43) Date of publication:
**05.02.2003 Bulletin 2003/06**

(51) Int Cl.⁷: **C07D 405/12**, A61K 31/38

(21) Application number: **01118682.2**

(22) Date of filing: **03.08.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **CHIESI FARMACEUTICI S.p.A.**
**43100 Parma (IT)**

(72) Inventors:
- **Delcanale, Maurizio**
  **43100 Parma (IT)**
- **Amari, Gabriele**
  **43100 Parma (IT)**
- **Armani, Elisabetta**
  **43100 Parma (IT)**
- **Civelli, Maurizio**
  **43100 Parma (IT)**
- **Galbiati, Elisabetta**
  **43100 Parma (IT)**

(74) Representative: **Minoja, Fabrizio, Dr.**
**Bianchetti Bracco Minoja S.r.l.**
**Via Rossini, 8**
**20122 Milano (IT)**

# (54) 2H-1-benzopyran derivatives, processes for their preparation and pharmaceutical compositions thereof

(57) 2H-1-benzopyran derivatives, processes for their preparation and use thereof for the preparation of pharmaceutical compositions for the prevention and treatment of postmenopausal pathologies.

EP 1 281 710 A1

## Description

The present invention relates to 2H-1-benzopyran derivatives, processes for their preparation and their pharmaceutical compositions.

More precisely, the invention relates to 2H-1-benzopyran derivatives of formula (I):

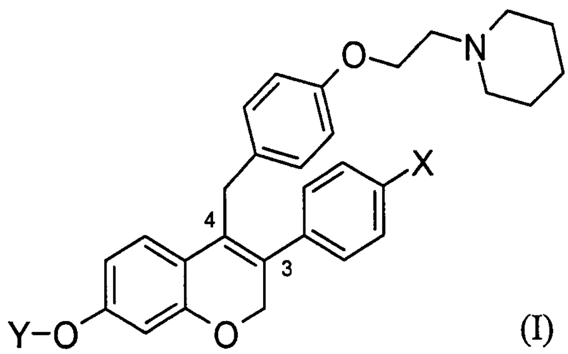

(I)

wherein X is $C_1$-$C_4$ alkoxy and Y is H, $C_1$-$C_4$ alkyl, $C_1$-$C_5$ alkanoyl, aryloyl.

Aryloyl is preferably benzoyl optionally substituted by one to three substituents, which are the same or different, selected from halogen atoms, $C_1$-$C_4$ alkyl, $C_1$-$C_4$ alkoxy, nitro, cyano, trifluoromethyl, amino, hydroxy groups.

The compounds can exist in the form of non-toxic organic or inorganic acid addition salts, or of addition salts with alkaline or alkaline-earth metal or with organic amines. The invention refers therefore also to the non-toxic salts of the compounds of formula (I) as well as the non-toxic eters thereof (when Y is H).

The compounds of formula (I) belong to the class of the Selective Estrogen Receptor Modulators (SERMs) which bind and interact with the estrogen receptor but which act in certain tissues, such as bone, as estrogen agonists and in other tissues, and in particular in the breast and uterus, as estrogen antagonists. The SERMs retain the beneficial effects of estrogen without some of its side effects.

## BACKGROUND OF THE INVENTION

WO 94/20098 in the name of Zymogenetics 3,4-diaryl-2H-1-benzopyrans have been disclosed for use in reducing bone loss and in particular bone loss associated with osteoporosis.

A particularly preferred compound according to the Zymogenetics invention is the 1-enantiomer of centchroman, later developed by Novo Nordisk as levormeloxifene for the treatment of osteoporosis.

Levormeloxifene is hereinafter used as reference compound.

Substituted benzopyran and thiobenzopyran derivatives having a potential anti-estrogenic activity have been later on disclosed in WO 99/65893 in the name of C&C Research Laboratories.

The compounds of the invention differ in their chemical structure from the know benzopyrans in:

- the presence of a double bond between the atoms in position 3-4
- the substitution on the carbon atom in 4 of the benzopyran ring with a aminoalkyloxybenzyl radical;

These differences in the benzopyran structure and in particular the presence of a methylene bridge between the carbon atom in position 4 of the benzopyran ring and the carbon atom of the phenyl group turned out to be of fundamental importance for the pharmacological activity of the compounds of the invention.

In the European Patent Application n. EP 01101521.1 in the Applicant's name 2H-1-benzopyran derivatives of formula (II)

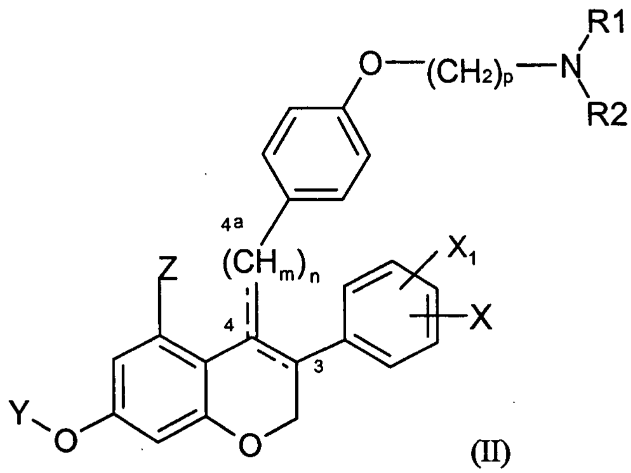

(II)

wherein

$R_1$ and $R_2$ independently of one another are H, alkyl, halogenoalkyl, alkenyl or halogenoalkenyl, or together with nitrogen atom to which they are bound can form a 4- to 8-membered heterocyclic ring
X is H, alkyl, aryl, nitro, halo, O-$R_3$ wherein $R_3$ is H, alkyl, aryl, alkanoyl, aryloyl
$X_1$ is H or alkyl and when X and $X_1$ are alkyl they can form together with the carbon atom they are bound to, a fused aromatic ring to give an $\alpha$-naphtalenyl
Y is H, alkyl, alkanoyl, aryloyl
Z is H, O-$R_4$ wherein $R_4$ is H, alkyl, alkanoyl, aryloyl
m is 1,2
n is 0,1
p is 2-6

------ represents a single or double bond between the atoms in positions 3-4 or 4-4a: when n is 1 the double bond may be alternatively exocyclic or endocyclic to give respectively a 4-benzylidenchroman for m=1 or a 4-benzyl-chrom-3-ene for m=2; when n is 0 the endocyclic bond 3-4 may be a single or a double bond and when n is 0 and the endocyclic bond 3-4 is a single bond, Z is not H have been described.

## DESCRIPTION OF THE INVENTION

It has now been found that among the compounds of formula (II) the insertion of a 4-alkoxy group, and preferably a 4-methoxy group, on the phenyl ring in position 3 of the benzopyran improved bone efficacy, serum cholesterol lowering, benefits in the uterus and oral bioavailability.
A non-steroidal benzothiophene derivative raloxifene, described in Jones CD et al J Med Chem 27, 1057-159, 1984, provided with tissue-specific estrogen agonist and antagonist actions has been admitted for clinical use in the USA and in some European countries.
The compounds of the invention favourably compare also with raloxifene.
Other synthetic compounds with this possible spectrum of activities, including triphenylethylene and dihydronaphthalene have been described as SERMs. However, the development of many of these compounds as drugs turned out to be problematic due to their excessive stimulation of uterine tissue.
The novel SERMs of the invention demonstrate significant beneficial effects on bone and serum lipid levels and antagonistic effects associated with a low degree of intrinsic estrogenicity on reproductive tissue.
For their tissue-specific estrogen-agonistic and antagonistic properties the compounds of the invention can be used in therapy in particular for the prevention and treatment of a number of postmenopausal pathologies, in particular osteoporosis, coronary heart disease and estrogen dependent human cancer.
Compounds of formula (I) and particularly the compound wherein Y is H and X is $OCH_3$ (formula (XI)) were obtained following scheme (1).
Compound (III) was protected at the phenolic hydroxy group with an appropriate protective moiety (Z) chosen between those described in the literature for the protection of phenols (see for examples T. W. Greene, P. G. Wuts

"Protective groups in organic synthesis", 3rd Ed, John Wiley & Sons, Inc., 1999, pages 246-292) to obtain compound (IV). The protective group must be chosen between those that are stable in the conditions of the next steps. The compound (IV) was subsequently hydrogenated selectively at the olefinic double bond, in a manner to avoid the reduction of ketone to alcohols, by means of a catalytic hydrogenation with an appropriate catalyst (containing Pd, Pt, Rh, Ru or other transition metals) in an appropriate solvent (alcohols, THF, acetone, dioxane, ethyl acetate, alcohols-water mixtures etc.) or by a chemical reduction with an hydride in an appropriate aprotic solvent, to give compound (V). Compound (V) was reacted with compound (VI) (obtained by reaction of protected 4-hydroxybenzylchloride with Mg in a suitable solvent like diethyl ether, THF or other ethereal solvents at temperature between r.t. and the boiling temperature of the solvent), so obtaining a compound of formula (VII). The protective group (Y') for 4-hydroxybenzylchloride must be chosen between the same previously described, but it must be different from Z selected for compound (IV). Selective removal of this second protective group (Y') gave compound (VIII). Compound (VIII) was alkylated with N-(2-haloethyl)piperidine in a suitable solvent and in presence of a base, to give compound (IX). Cleavage of the first introduced protective group (Z) gave compound (X). Compound (X) was dehydrated (for example with an acid in an appropriate solvent) to give compound (XI). With an appropriate choice of Z it is possible to obtain compound (XI) directly from compound (IX) by acidic removal of Z protective group and, simultaneously, hydrolysis of the tertiary alcohol.

Scheme 1

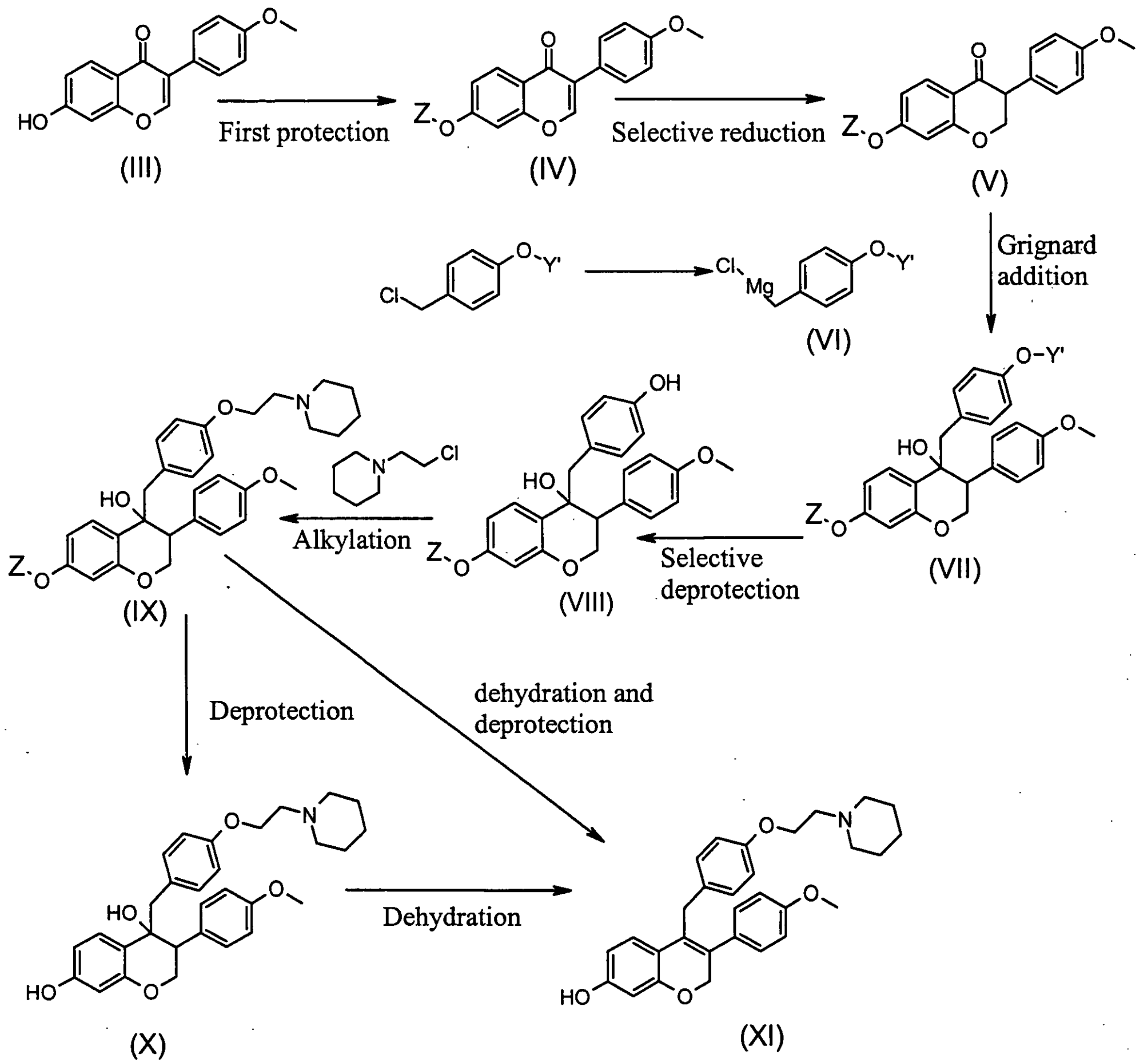


The following example further illustrates the invention.

**Example 1: Synthesis of 3-(4-metoxy)phenyl-4-[[4-[2-(1-piperidinyl) ethoxy]phenyl]methyl]-2H-1-benzopyran-7-ol hydrochloride (Compound IX)**

a) Synthesis of 2,2-dimethyl-propanoic acid 3-(4-methoxyphenyl)-4-oxo-4H-1-benzopyran-7-yl ester ( Compound (IV) wherein Z=$(CH_3)_3CCO$-)

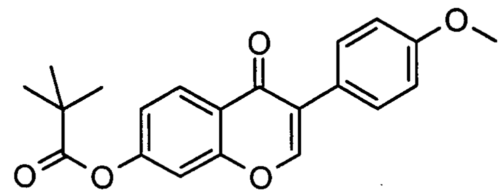

Formononetine (compound III) (90g; 335 mmol) was suspended in acetonitrile, $K_2CO_3$ was added and the mixture was stirred for about 20 minutes at r.t.. Pivaloyl chloride (59.85 g; 496 mmol) was dropped into the mixture, then the reaction was stirred for further 15 minutes and quenched into water. The solid was filtered, washed with water, dissolved in $CHCl_3$; the solution was dried and evaporated to dryness to obtain the title compound, which can be crystallised from hot toluene. 102 g of a white crystalline powder were obtained.

M.P.: 159-161° C; NMR, MS and IR comply with the structure.

b) Synthesis of 2,2-dimethyl-propanoic acid 3,4-dihydro-3-(4-methoxy phenyl) -4-oxo-2H-1-benzopyran-7-yl ester (Compound (V) wherein Z=$(CH_3)_3CCO$-)

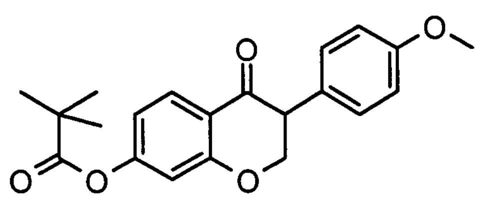

Compound (IV) wherein Z=$(CH_3)_3CCO$- (80 g; 227 mmol) was dissolved in dioxane and hydrogenated in a Parr apparatus at 35 psi with Pd/$BaSO_4$ 5% as catalyst.

After 3 hours the catalyst was filtered off, the solution was evaporated to a little volume and quenched into water to obtain a precipitate that was filtered and dissolved in hot 95% ethanol. After standing overnight in a refrigerator, the white product was filtered and washed with petroleum ether, so obtaining 66 g of the title compound.

M.P.: 119-121° C; NMR, MS and IR comply with the structure.

c) Synthesis of 2,2-dimethyl-propanoic acid 3,4-dihydro-4-hydroxy-3-(4-methoxyphenyl)-4-[[4-(phenylmethoxy) phenyl]methyl]-2H-1-benzopyran-7-yl ester (Compound (VII) wherein Z=$(CH_3)_3CCO$- and Y'=$C_6H_5$-$CH_2$)

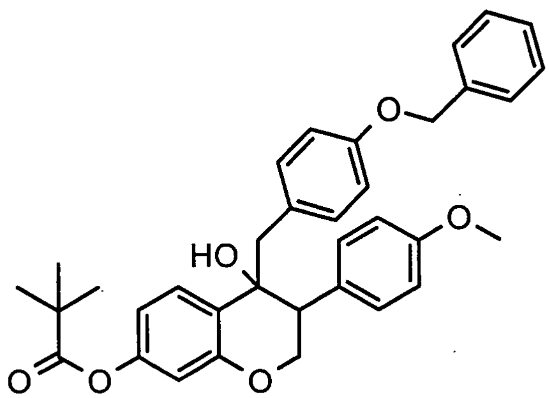

Mg turnings (15.4 g, 635 mmol) were loaded into a 2 liter flask; 4-benzyloxybenzyl chloride (49.6 g, 211.6 mmol) was dissolved in dry THF and this solution was dropped in one hour on the Mg turnings under an inert atmosphere while maintaining a gentle reflux.

The mixture was refrigerated to -20°C, then a solution of compound (V) wherein Z=$(CH_3)_3CCO$- (60 g, 169.3 mmol) in dry THF (300 ml) was dropped in a 15 minutes period. After leaving the mixture to reach r.t., water (50 ml) was added, the solid material was filtered off and the solution was evaporated to dryness. Ethyl acetate (200 ml) was added to the residue oil and the mixture was allowed to stay at -20°C for one hour. The solid was filtered off and the solution was evaporated to dryness. The residue oil was crystallised from a 80/20 mixture of 95% ethanol and petroleum ether to give 60.8 g (110 mmol) of compound (VII) wherein Z=$(CH_3)_3CCO$- and Y'= $C_6H_5$-$CH_2$.

M.P.: 104-111° C; NMR, MS and IR comply with the structure.

d) Synthesis of 2,2-dimethyl-propanoic acid 3,4-dihydro-4-hydroxy-4-[(4-hydroxyphenyl)methyl]-3-(4-methoxyphenyl)-2H-1-benzopyran-7-yl ester (Compound (VIII) wherein Z=$(CH_3)_3CCO$-)

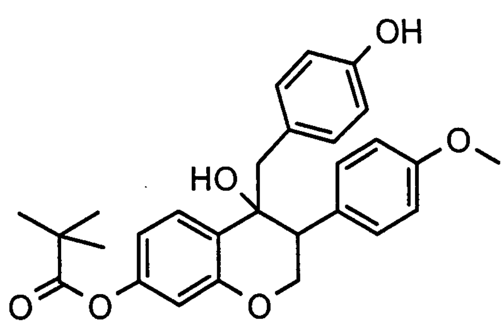

Compound (VII) wherein Z=$(CH_3)_3CCO$- and Y'=$C_6H_5$-$CH_2$ (60.8 g, 110 mmol) was dissolved in ethyl acetate, 5% Pd/C was added and the mixture was hydrogenated.

The catalyst was filtered off and the solution was evaporated to dryness obtaining a solid foam (50.88 g, 110 mmol) that was used without any further purification.

NMR, MS and IR comply with the structure.

e) Synthesis of 2,2-dimethyl-propanoic acid 3,4-dihydro-4-hydroxy-3-(4-methoxyphenyl)-4-[[4-[2-(1-piperidinyl) ethoxy]phenyl]methyl]-2H-1-benzopyran-7-yl ester (Compound (IX) wherein Z=$(CH_3)_3CCO$-)

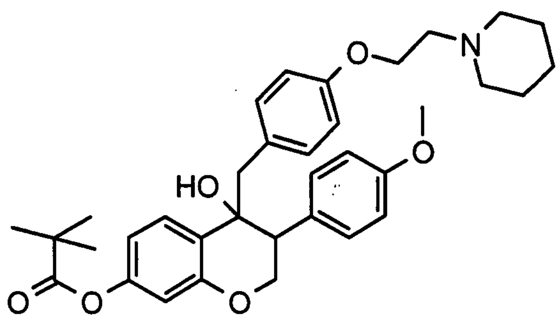

A mixture of: compound (VIII) wherein Z=$(CH_3)_3CCO$- (50 g, 108.1 mmol), N-(2-chloroethyl)piperidine hydrochloride (20 g,108.7 mmol) and K2CO3 in boiling acetone (860 ml) was allowed to react.

The solid was filtered off and the solution was evaporated to dryness obtaining an oil (62 g, 108.1 mmol) that was used without any further purification.

NMR, MS and IR comply with the structure.

f) Synthesis of 3,4-dihydro-4-hydroxy-3-(4-methoxyphenyl)-4-[[4-[2-(1-piperidinyl)ethoxy]phenyl]methyl]-2H-1-benzopyran-4,7-diol (Compound (X))

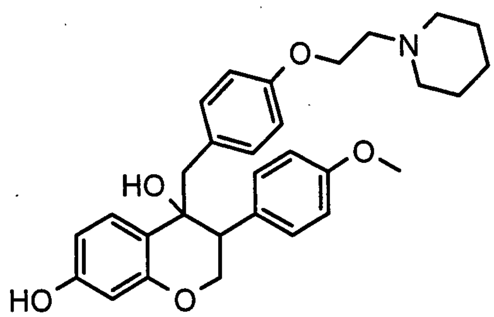

To a solution of compound (IX) wherein Z=$(CH_3)_3CCO$- (60 g, 104.6 mmol) in methanol, $K_2CO_3$ dissolved in water was added and allowed to react at r.t. for 3 hours. The solvent was evaporated to 300 ml and the mixture was quenched into water and stirred overnight. The solid was filtered, washed with water and dried under vacuum at r.t. obtaining 36 g of crude product that was crystallised from boiling acetonitrile to obtain 28 g of compound (X).

M.P.: 107.5-108.5° C; NMR, MS and IR comply with the structure.

g) Synthesis of 3-(4-metoxy)phenyl-4-[[4-[2-(1-piperidinyl)ethoxy]-phenyl]methyl]-2H-1-benzopyran-7-ol hydrochloride (Compound (XI) hydrochloride)

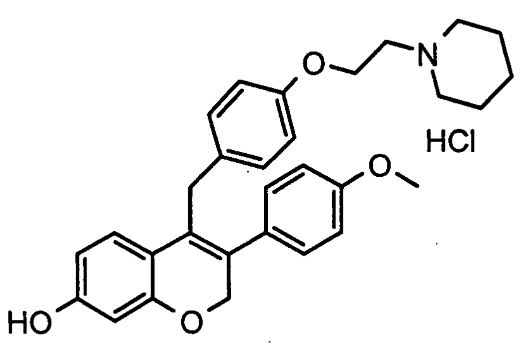

Compound (X) (24 g, 49 mmol) was dissolved in hot acetonitrile (550 ml), 37% HCl (4.9 ml) was added and the solution was stirred at reflux for 45 minutes. The mixture was allowed to reach r.t. and the crystallised solid

was filtered and washed with diethyl ether. 17.3 g (34 mmol) of the title compound were obtained.

M.P.: 199-205° C (dec.); NMR, MS and IR comply with the structure.

The compounds of the invention have been tested for their pharmacological activity.

In the following examples the results obtained with the preferred compound 3-(4-metoxy)phenyl-4-[[4-[2-(1-piperidinyl)ethoxy]phenyl]-methyl]-2H-1-benzopyran-7-ol hydrochloride (the compound of example 1) are reported.

**In vitro study - Human estrogen receptor-α and -β (ER-α and ER-β)**

The experiment has been conducted as described in Obourn JD et al, Biochemistry 32, 6229, 1993.

The compound of the invention binds with high affinity to the human estrogen receptor ER-α and ER-β. Binding $K_i$ were 0.017 ± 0.002 and 0.099 ± 0.005 nM, respectively.

Compared with the well known SERM raloxifene ($K_i$ ER-β = 1.62 ± 0.348, ER-α = 0.071 ± 0.008 nM), the affinity for ERβ was 16 fold higher, whereas the affinity for ER-α was similar.

**In vivo studies - Immature female rat assay.**

Antiuterotrophic activity

The experiment has been conducted as described in Eppenberger U et al, Am J Clin Oncol 14 (suppl. 2), S5-S14, 1991.

In immature female rats, treatment with 17α-ethynil estradiol (E2) at 0.05 mg/kg p.o. for 3 days significantly increased uterine wet weight (about 170 %) compared with vehicle-treated controls.

This concentration of E2 was the lowest producing near-maximal effect and was chosen on the basis of preliminary dose-response experiments.

The compound of the invention administered orally inhibited the uterotrophic action of E2 in a dose related fashion, and near-total antagonism was observed at 0.1 mg/kg·day.

Comparison of the antiestrogenic potencies (ED50 = dose required to produce 50% reduction in estradiol-stimulated growth) showed that the compound of the invention was about 25 times more potent than raloxifene (ED50 = 0.016 and 0.39 mg/kg·day, respectively).

Uterotrophic activity

Uterine weights for 0.01, 0.1, 1 and 10 mg/kg·day of the compound of example 1 and raloxifene were not significantly different from control rats. At the same dose levels, levormeloxifene significantly increased uterine wet weight in a dose-depend way and a maximal agonist activity of 55-65 % that of E2 was apparent at 1 mg/kg.

We can conclude that in the immature female rat assay, the preferred compound of the invention did not induce agonistic effects on uterine tissue, producing total antagonism of E2-induced uterine growth and appeared more potent than raloxifene in antagonizing estrogen stimulation of the uterus.

**Effects in the ovariectomized (OVX) rat model**

The experiments have been conducted according to Kalu DN, Bone Mineral 15, 175-192, 1991 and Grese TA et al J Med Chem 40, 146-167, 1997.

Four-week OVX rat assay

The effects of the tested compound was evaluated in 9-11-month-old OVX rats that were dosed for 4 weeks post-surgery and compared with OVX and Sham controls.

Tissue-specific estrogen agonist effects were examined utilizing uterine weight, uterine eosinophil peroxidase activity (EPO), bone mineral density (BMD), and serum cholesterol levels as end points.

The administration for 4 weeks was not associated with any overt signs of toxicity.

Effect on bone mineral density (BMD) (lumbar spine L1-4) and serum ostecalcin

Bone mineral density (BMD) was measured by DEXA (Dual Energy XRay Absorptiometry) using a Hologic QDR-1000 plus instrument equipped with dedicated software for small animal measurements. An ultrahigh-resolution mode (line spacing 0.0254 cm and resolution 0.0127 cm) was used with a collimator of 0.63 mm diameter. This technique provides an integrated measure of both cortical and trabecular bone.

In vivo DEXA measurements were carried out immediately before surgery (baseline scan) and 4 weeks after surgery. The anatomic region examined was the lumbar spine L1-4.

All animals were anesthetized before scanning with a mixture of ketamine and p-promazine. For each scan a rat was placed in a supine position with the spine parallel to the long axis of the densitometer table. The lumbar spine was scanned using the pelvic bones as landmark; analysis of this site was accomplished by dividing vertebra and intervertebral spaces with subregional high resolution software and including only target vertebra in the global region of interest.

Percent protection was calculated by the following formula: % protection =

$$[(\%\ \text{change BMD}_{\text{test compound}} - \%\ \text{change BMD}_{\text{OVX control}})/(\%\ \text{change BMD}_{\text{sham control}} - \%\ \text{change BMD}_{\text{OVX control}})]\text{x}100.$$

4 weeks after surgery a significantly higher bone loss from baseline in OVX rats compared to sham rats was observed (% change in BMD -5.16 % and +1.28 %, respectively $P < 0.01$).

As shown in the literature, 0.1 mg/kg of 17α-ethynil estradiol (E2) partially prevented the loss of bone (~95% protection).

0.1 and 1 mg/kg·day of the tested compound treatment reduced the bone loss in a dose dependent manner, as their % change in BMD from the baseline was significantly higher compared to OVX rats (60 and 119% protection, respectively, $P<0.01$).

The bone sparing effects of the tested compound was comparable with those achieved with E2 0.1 mg/kg·day, indicating that it is a full estrogen agonist on bone.

The protective effect of raloxifene and levormeloxifene was significant (50-60 % protection at 1 and 10 mg/kg·day) but significantly lower compared to the one of the compound of the invention.

<u>Effect on serum cholesterol levels</u>

0.1 and 1 mg/kg·day of the compound of the invention dose-dependently decreased total serum cholesterol in OVX rats (75 and 81% inhibition respectively) The compound of the invention was as efficacious as E2 in lowering serum cholesterol levels with respect to controls, whereas raloxifene and levormeloxifene appeared significantly less active.

<u>Uterine effects</u>

In OVX rats treated with the compound of the invention at 0.1 and 1 mg/kg·day, uterine weight was significantly lower than both sham controls and E2-treated OVX rats.

Moreover the compound of the invention had no significant effects on eosinophilic peroxidase activity (a useful marker for estrogen-effected growth responses, Lyttle CR and DeSombre ER Proc Natl Acad Sci USA 74, 3162-3166, 1977)

We can conclude that the compound of the invention administered at 0.1 and 1 mg/kg·day for 4 weeks by oral route, significantly reduced bone loss while lowering serum cholesterol levels.

These protective effects were achieved at a dose with minimal uterine stimulation.

Taken together, the reported results show that the benzopyran derivatives of the invention are provided with an interesting SERM (Selective Estrogen Receptor Modulation) activity, comparing favorably with the structurally related reference compounds of the prior art.

In view of their therapeutic use, the compounds of the invention can be opportunely combined with the usual pharmaceutically acceptable excipients for the preparation of pharmaceutical compositions for parenteral, oral, nasal, rectal, subdermal or transdermal administration according to conventional methods.

One skilled in this art may formulate the compounds in an appropriate manner, and in accordance with accepted practices, such as those disclosed in *Remington's Pharmaceutical Sciences,* Gennaro ed., Mack Publishing Co., Easton, PA, 1990.

## Claims

**1.** 2H-1-benzopyran derivatives of general formula:

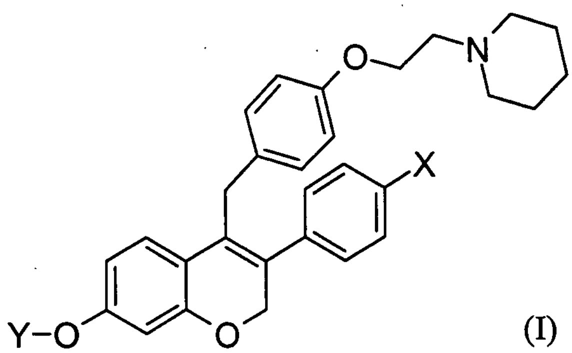

(I)

wherein X is $C_1$-$C_4$ alkoxy and Y is H, $C_1$-$C_5$ alkanoyl, aryloyl.

**2.** The compound according to claim 1 that is 3-(4-methoxy)phenyl-4-[[4-[2-(1-piperidinyl)ethoxy]phenyl]-methyl]-2H-1-benzopyran-7-ol, non-toxic esters and salts thereof.

**3.** Pharmaceutical compositions containing as the active ingredient at least one compound as claimed in claims 1 and 2 together with pharmaceutically acceptable excipients.

**4.** The use of the compounds according to claims 1-2 for the preparation of a medicament useful in the prevention and treatment of a number of postmenopausal pathologies, in particular osteoporosis, coronary heart disease and estrogen dependent human cancer.

European Patent Office

# EUROPEAN SEARCH REPORT

Application Number
EP 01 11 8682

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | WO 98 25916 A (KANBE YOSHITAKE ;MORIKAWA KAZUMI (JP); NISHIMOTO MASAHIRO (JP); JO) 18 June 1998 (1998-06-18) * claim 1 * | 1-4 | C07D405/12 A61K31/38 |
| Y | US 5 280 040 A (LABROO VIRENDER M ET AL) 18 January 1994 (1994-01-18) * column 2, line 38 - column 3, line 4 * | 1-4 | |
| Y | US 5 985 306 A (BURY PAUL STANLEY ET AL) 16 November 1999 (1999-11-16) * column 5, line 60 - column 6, line 16 * | 1-4 | |
| Y | WO 01 49673 A (SIGNAL PHARM INC) 12 July 2001 (2001-07-12) * claim 1 * | 1-4 | |
| Y | WO 00 39120 A (BHAGWAT SHRIPAD S ;KHAMMUNGKHUNE SAK (US); SHEVLIN GRAZIELLA I (US) 6 July 2000 (2000-07-06) * claim 1 * | 1-4 | |

TECHNICAL FIELDS SEARCHED (Int.Cl.7)
C07D
A61K

The present search report has been drawn up for all claims

| Place of search | Date of completion of the search | Examiner |
|---|---|---|
| MUNICH | 6 November 2001 | Samsam Bakhtiary, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 01 11 8682

This annex lists the patent family members relating to the patent documents cited in the above-mentioned European search report.
The members are as contained in the European Patent Office EDP file on
The European Patent Office is in no way liable for these particulars which are merely given for the purpose of information.

06-11-2001

| Patent document cited in search report | | Publication date | Patent family member(s) | | | Publication date |
|---|---|---|---|---|---|---|
| WO 9825916 | A | 18-06-1998 | AU | 722089 | B2 | 20-07-2000 |
| | | | AU | 5413498 | A | 03-07-1998 |
| | | | CN | 1244863 | A | 16-02-2000 |
| | | | EP | 0944613 | A1 | 29-09-1999 |
| | | | JP | 2000507620 | T | 20-06-2000 |
| | | | WO | 9825916 | A1 | 18-06-1998 |
| | | | US | 6153768 | A | 28-11-2000 |
| US 5280040 | A | 18-01-1994 | AT | 178488 | T | 15-04-1999 |
| | | | AU | 695497 | B2 | 13-08-1998 |
| | | | AU | 1639497 | A | 22-05-1997 |
| | | | AU | 674394 | B2 | 19-12-1996 |
| | | | AU | 6230294 | A | 26-09-1994 |
| | | | BR | 9405843 | A | 16-01-1996 |
| | | | CA | 2157879 | A1 | 12-09-1994 |
| | | | CZ | 9502320 | A3 | 17-04-1996 |
| | | | DE | 69417733 | D1 | 12-05-1999 |
| | | | DE | 69417733 | T2 | 16-09-1999 |
| | | | DK | 688214 | T3 | 09-10-2000 |
| | | | EP | 0688214 | A1 | 27-12-1995 |
| | | | ES | 2131678 | T3 | 01-08-1999 |
| | | | GR | 3030128 | T3 | 30-07-1999 |
| | | | HU | 74575 | A2 | 28-01-1997 |
| | | | JP | 2834581 | B2 | 09-12-1998 |
| | | | JP | 8506346 | T | 09-07-1996 |
| | | | KR | 263009 | B1 | 01-08-2000 |
| | | | NO | 953542 | A | 08-09-1995 |
| | | | NZ | 262569 | A | 22-08-1997 |
| | | | SG | 65584 | A1 | 22-06-1999 |
| | | | WO | 9420098 | A1 | 15-09-1994 |
| | | | US | 5464862 | A | 07-11-1995 |
| US 5985306 | A | 16-11-1999 | AU | 4771997 | A | 22-05-1998 |
| | | | WO | 9818775 | A1 | 07-05-1998 |
| | | | EP | 0934298 | A1 | 11-08-1999 |
| | | | JP | 2001502707 | T | 27-02-2001 |
| | | | NO | 992008 | A | 25-06-1999 |
| WO 0149673 | A | 12-07-2001 | US | 6291456 | B1 | 18-09-2001 |
| | | | AU | 3077101 | A | 16-07-2001 |
| | | | WO | 0149673 | A2 | 12-07-2001 |
| WO 0039120 | A | 06-07-2000 | AU | 2597000 | A | 31-07-2000 |
| | | | EP | 1140889 | A2 | 10-10-2001 |
| | | | WO | 0039120 | A2 | 06-07-2000 |
| | | | US | 6291456 | B1 | 18-09-2001 |

EPO FORM P0459

For more details about this annex : see Official Journal of the European Patent Office, No. 12/82